Europäisches Patentamt

(19) European Patent Office

Office européen des brevets

(11) **EP 0 542 969 B1**

(12) **FASCICULE DE BREVET EUROPEEN**

(45) Date de publication et mention
de la délivrance du brevet:
**07.05.1997 Bulletin 1997/19**

(21) Numéro de dépôt: **92911799.2**

(22) Date de dépôt: **04.06.1992**

(51) Int. Cl.$^6$: **A61K 9/50**, A61K 9/51,
A61K 9/54, A61K 47/48

(86) Numéro de dépôt international:
**PCT/FR92/00498**

(87) Numéro de publication internationale:
**WO 92/21329 (10.12.1992 Gazette 1992/31)**

(54) **VECTEUR PARTICULAIRE BIODEGRADABLE ET PROCEDE DE SYNTHESE**

BIOLOGISCH ABBAUBARER PARTIKELFÖRMIGER VEKTOR UND SYNTHESEVERFAHREN

BIODEGRADABLE PARTICULATE VECTOR AND METHOD OF SYNTHESIS

(84) Etats contractants désignés:
**AT BE CH DE DK ES FR GB GR IT LI LU NL SE**

(30) Priorité: **04.06.1991 FR 9106743**

(43) Date de publication de la demande:
**26.05.1993 Bulletin 1993/21**

(73) Titulaire: **BIOVECTOR THERAPEUTICS SA**
**F-31520 Ramonville-Saint-Agne (FR)**

(72) Inventeurs:
 • **SAMAIN, Daniel**
 **F-31400 Toulouse (FR)**
 • **DE MIGUEL, Ignacio**
 **F-31400 Toulouse (FR)**
 • **MENIALI, Jaouad**
 **F-31400 Toulouse (FR)**
 • **IOUALALEN, Karim**
 **F-31500 Toulouse (FR)**

 • **DING, Li**
 **F-31400 Toulouse (FR)**
 • **CERVILLA, Monique**
 **F-31400 Toulouse (FR)**
 • **RIEUMAJOU, Valérie**
 **F-47220 Astaffort (FR)**
 • **DELRIEU, Pascal**
 **F-31400 Toulouse (FR)**
 • **IMBERTIE, Laurent**
 **125, avenue Jules-Julien**
 **F-31400 Toulouse (FR)**

(74) Mandataire: **Warcoin, Jacques**
 **Cabinet Régimbeau,**
 **26, avenue Kléber**
 **75116 Paris (FR)**

(56) Documents cités:
 **EP-A- 0 344 040          EP-A- 0 397 227**

EP 0 542 969 B1

## Description

La présente invention concerne un vecteur particulaire, biodégradable, utile pour le transport de molécules à activité biologique.

Elle concerne également un procédé de synthèse de ce vecteur, et d'encapsulation de principes actifs dans ce vecteur.

De tels vecteurs constituent l'un des procédés utilisés pour faire pénétrer ou réagir un composé actif à l'intérieur d'un système biologique ou biochimique. En effet, ils doivent permettrent en encapsulant un tel composé, de la protéger vis-à-vis des agents normaux de son catabolisme, et de l'amener jusqu'à son lieu d'action, où le vecteur le libérera.

On a utilisé dans ce but des liposomes, qui sont constitués d'une vacuole aqueuse entourée d'une bicouche phospholipidique. Un tel système de transport présente cependant un certain nombre de limitations dues à la fragilité des liposomes, leur hétérogénéité et la complexité de leur production à l'échelle industrielle. En outre, leur capacité de transport est réduite.

Une autre solution consiste à utiliser des biovecteurs supra-moléculaires (ou BVSM), tels qu'ils sont décrits dans le brevet EP- 344 040.

De tels vecteurs comprennent un noyau central de polysaccharides réticulés, entouré d'une première couche lipidique liée au noyau par des liaisons covalentes, et d'une seconde couche ou feuillet externe de composés amphiphiles.

Ces biovecteurs sont très stables, lyophilisables, stérilisables, et leur structure présente de fortes analogies avec les vecteurs naturels.

Ces biovecteurs peuvent encapsuler des principes actifs de natures chimiques différentes, en accord avec les caractéristiques des trois zones : un noyau polaire, une couronne lipidique et un feuillet externe amphiphile.

Toutefois, ces vecteurs présentent certaines imperfections. En effet, le taux de principe actif pouvant être encapsulé reste relativement faible. En outre, le principe actif doit être encapsulé au moment de la synthèse de la zone du biovecteur correspondante, et, pour les principes actifs encapsulés notamment dans le noyau, il y a un risque d'altération lors de la synthèse des couches suivantes.

EP- 397 227 décrit un procédé de préparation de produits particulaires antimicrobiens, par ancrage de l'enzyme lactopéroxydase sur la première couche lipidique et/ou la seconde couche phospholipidique d'un vecteur particulaire, synthétisé en milieu hydrophobe.

Il est donc souhaitable de disposer de vecteurs particulaires permettant le chargement de principes actifs avec des rendements quantitatifs, selon un protocole permettant la conservation de l'intégrité structurale des principes actifs, ces vecteurs restant cependant parfaitement biodégradables et biocompatibles avec l'organisme.

C'est pourquoi la présente invention a pour objet un vecteur particulaire biodégradable, caractérisé en ce qu'il comporte :

- un noyau constitue d'une matrice polysaccharidique ou oligosaccharidique réticulée, sur laquelle sont gréffés des ligands ioniques,
- une première couche lipidique semi-perméable, liée au noyau par des liaisons covalentes,
- une seconde couche de composés amphiphiles, liée à la première couche lipidique par des interactions hydrophobes.

Un tel vecteur permet l'encapsulation optimisée de principes actifs à l'intérieur du noyau polysaccharidique.

Ces propriétés sont obtenues par le greffage d'un grand nombre de ligands ioniques, hydrolysables enzymatiquement, sur la matrice réticulée. Le noyau, auquel est ainsi conféré un caractère ionique important, est doté alors de propriétés d'encapsulation très supérieures à celles observées pour les vecteurs particulaires de l'art antérieur, et il est complètement biocompatible par le choix adapté de ces ligands et des constituants de la matrice.

Le greffage ionique ne doit pas être préjudiciable à la stabilité ni à la taille de la matrice polysaccharidique. Ces ligands seront donc choisis de préférence parmi des molécules biologiques naturellement présentes dans l'organisme, et couplés à la matrice par des liaisons hydrolysables enzymatiquement.

Selon un des aspects de l'invention, les ligands greffés sur la matrice sont des composés acides. Ces ligands acides peuvent notamment être choisis parmi l'acide succinique, l'acide phosphorique, l'acide citrique, la glycine, l'alanine, l'acide glutamique, l'acide aspartique.

L'acide succinique est un constituant naturel de l'organisme et intervient dans le cycle de Krebs. C'est un diacide carboxylique qui peut former avec les hydroxyles de la matrice polysaccharidique une liaison ester facilement biodégradable grâce à la présence ubiquitaire dans l'organisme de succinylestérase. La fonction acide qui n'est pas engagée dans la fontion ester avec la matrice polysaccharidique reste donc disponible pour assurer les propriétés d'échange d'ions.

Les phosphates sont également des fonctions extrêmement fréquentes dans l'organisme. On les retrouve principalement associés à des lipides, des sucres et à des nucléotides.

On peut également greffer sur la matrice des mono hydroxy ou des monoaminoacides, par exemple l'acide citrique, les acides amines a chaîne courte et les acides aminés acides.

Il est intéressant de disposer de noyaux ayant un taux de greffage de ligands suffisant pour permettre un niveau d'encapsulation ultérieur satisfaisant. Ce taux doit se rapprocher d'une charge par sucre élémentaire.

On obtient par exemple des vecteurs particulaires dans lesquels l'acide succinique est greffé à un taux correspondant environ à une charge pour 1,5 résidus de glucose ; ou bien l'acide phosphorique est greffé à un taux correspondant environ à une charge pour 1,5 résidus de glucose.

Selon un autre aspect de l'invention, les vecteurs particulaires sont caractérisés en ce que les ligands greffés sur la matrice sont des composés basiques, fixés à la matrice par l'intermédiaire d'un composé acide.

Afin d'obtenir le greffage biodégradable de ligands basiques, la Demanderesse a trouvé qu'il était possible d'utiliser notamment l'acide succinique entre la matrice polysaccharidique et un composé basique. L'une des fonctions acides de l'acide succinique est utilisée pour réaliser une fonction ester biodégradable avec la matrice polysaccharidique et l'autre fonction acide est utilisée pour réaliser une fonction ester ou amide avec le composé basique.

De préférence, les ligands basiques greffés sur la matrice sont des composés bifonctionnels comportant une fonction acylable formant une liaison amide ou ester avec un composé acide fixe sur la matrice.

La fonction acylable est par exemple une fonction hydroxyle, amine primaire ou secondaire.

L'autre fonction, basique, non acylable, est par exemple une fonction amine tertiaire ou quaternaire.

Le ligand basique peut notamment être choisi parmi, la choline, l'hydroxycholine, le 2-(diméthylamino) éthanol et la 2-(diméthylamino)éthylamine.

Des taux de greffage ionique correspondant à une charge pour trois résidus glucose peuvent être observés.

La matrice polysaccharidique, elle-même biodégradable, peut être constituée d'un polysaccharide réticulé chimiquement choisi parmi le dextrane, l'amidon, la cellulose, les oligosaccharides et leurs dérivés.

Elle peut être notamment réalisée en réticulant un polysaccharide biodégradable comme l'amidon avec un agent bifonctionnel, comme l'épichlorhydrine. Dans le cas où le rapport épichlorhydrine/glucose est maintenu en dessous de 1/10, le gel obtenu conserve la propriété d'être hydrolysé par les amylases. La cinétique d'hydrolyse est toutefois ralentie lorsque ce rapport augmente et se rapproche de 1/10. L'analyse des produits d'hydrolyse enzymatique des matrices d'amidon réticulé ne permet pas de mettre en évidence des oligomères de glucose non hydrolysable de taille supérieure à 10. L'administration de telles matrices d'amidon réticulé à l'intérieur de l'organisme ne doit donc pas conduire à la formation de polymères de glucose non éliminables par l'organisme.

Il est aussi possible de réticuler un oligo- ou polysaccharide biodégradable par réaction directe avec l'oxychlorure de phosphore ou un dérivé de l'acide phosphorique. La Demanderesse a trouvé qu'il est possible d'obtenir des matrices polysaccharidiques phosphorylées grâce à la formation des liaisons phosphodiester. Dans ce cas, la matrice est directement dérivée avec des charges négatives, par les fonctions acides du phosphate formé.

Il est également possible de coréticuler la matrice polysaccharidique ou oligosaccharidique avec des composés tels que des protéines ou des peptides. Parmi les protéines utilisables, il faut citer : la kératine, le collagène, l'élastase, leurs dérivés et leurs analogues.

Les noyaux des vecteurs particulaires selon l'invention possèdent une certaine porosité, qui dépend des conditions de réticulation, de la nature du ligand ionique et de son taux de greffage.

La première couche lipidique, semi-perméable, des vecteurs particulaires selon l'invention est de préférence constituée d'acides gras naturels, fixés à un taux variable. En effet, la densité de cette couche lipidique peut être modulée par une acylation ménagée. Le contrôle de la réaction d'acylation peut être réalisé soit par le contrôle de la stoéchiométrie des réactifs, soit par le contrôle de la cinétique réactionnelle. Les couches lipidiques peu denses rendent les particules seulement partiellement hydrophobes. Il en résulte que les particules possèdent notamment la capacité de s'hydrater partiellement. La présence des acides gras placés en périphérie ne permet cependant pas aux particules de se disperser librement dans les milieux aqueux et les noyaux faiblement acylés sont observés sous forme d'agrégats dont la cohésion est due à des liaisons de type hydrophobe.

Dans un mode de réalisation préférée, le vecteur particulaire selon l'invention peut être caractérisé en ce que la seconde couche lipidique est constituée de phospholipides, ou de céramides.

Les particules sont alors à nouveau complètement dispersées. Toutefois, le rapport optimal pour atteindre une dispersion maximale entre le poids de noyau acylé et le poids de phospholipides est plus important dans le cas de noyaux ayant une première couche semi-perméable que dans le cas de noyaux complètement hydrophobes.

Il est possible qu'il se forme autour du noyau acylé un double feuillet de phospholipides, le feuillet interne étant interdigité par les acides gras de la première couche lipidique.

Un vecteur particulaire selon l'invention peut être caractérisé en ce qu'une molécule à activité biologique est incluse dans le noyau.

En effet, un vecteur selon l'invention peut encapsuler un principe actif, en mettant à profit la porosité du noyau. Ce noyau a de préférence une taille comprise entre 10 nm et 10 μm.

Les principes actifs peuvent pénétrer à l'intérieur du noyau polysaccharidique ionique au travers de la couche semi-perméable. Ils sont ensuite stabilisés et maintenus à l'intérieur du noyau par l'établissement de liaisons coulombiennes entre leurs charges et les charges de la matrice polysaccharidique.

De manière préférée, la molécule à activité biologique incluse dans le vecteur particulaire selon l'invention à un poids moléculaire compris entre 100 daltons et 500 kilodaltons.

La présente invention a également pour objet un procédé de synthèse d'un vecteur particulaire possédant une ou plusieurs des caractéristiques précédentes, ce procédé étant caractérisé en ce que :

a) on prépare une matrice par réticulation d'un polymère ou oligomère hydrophile biodégradable,

b) on fixe sur la matrice, des ligands ioniques par des liaisons hydrolysables enzymatiquement, afin d'obtenir le noyau du vecteur,

c) on soumet les noyaux à ultrabroyage pour les amener à une taille comprise entre 10 nm et 10 $\mu$m,

d) on sèche les noyaux,

e) on couple chimiquement sur les fonctions réactives à la surface du noyau des composés lipidiques pour former la première couche,

f) on introduit des composés amphiphiles en contact hydrophobe avec la première couche afin de former la seconde couche.

La réticulation de polymère hydrophile, notamment de polysaccharide, est un procédé connu de l'homme du métier. Toutefois, les conditions doivent être fixées pour obtenir une matrice qui reste biodégradable et, de préférence, ne donne pas naissance à des produits d'hydrolyse enzymatique constitué par des oligomères de glucose non hydrolysables de taille supérieure à 10.

Le greffage des ligands ioniques doit permettre d'obtenir un taux suffisant pour conferer un caractère ionique au noyau obtenu.

Dans le cas de l'acide succinique, les meilleurs résultats pour le greffage de l'acide succinique sont obtenus en utilisant le monochlorure de l'acide succinique. Une méthode originale de synthèse de ce réactif a été mise au point, utilisant une réaction de transchloration entre le dichlorure de l'acide succinique et l'acide succinique libre selon l'équation suivante :

$$ClCO\text{-}CH_2\text{-}CH_2\text{-}COCl + HOCO\text{-}CH_2\text{-}CH_2\text{-}COOH \rightarrow 2\ ClCO\text{-}CH_2\text{-}CH_2\text{-}COOH$$

Cette réaction est simple à effectuer et conduit avec un bon rendement au monochlorure pur et cristallisé.

L'intérêt de ce réactif par rapport aux autres réactifs déjà décrits est :

1) sa grande facilité de préparation

2) sa très grande réactivité chimique notamment vis-a-vis des hydroxyles

3) sa solubilité dans l'eau

4) l'absence de résidus ou de sous produits de réaction toxiques

La Demanderesse a trouvé que l'utilisation de ce réactif permettait le greffage d'acide succinique sur des matrices polysaccharidiques (PS) avec de bons rendements et dans des conditions réactionnelles très douces (0°C, pH 6,5). Des taux de greffage ionique correspondant à une charge pour 1,5 résidus de glucose peuvent notamment être atteints.

$$PS\text{-}OH + ClCO\text{-}(CH_2)_2\text{-}COOH \xrightarrow[0°C,pH6,5]{} PS\text{-}O\text{-}CO\text{-}(CH_2)_2\text{-}COOH$$

Cette réaction permet l'obtention d'une répartition régulière des ligands greffés sur la matrice polysaccharidique, contrairement à ce qui est observé par les techniques classiques, avec l'emploi d'anhydride succinique par exemple.

Le couplage de phosphates sur les matrices PS fait intervenir classiquement la réaction de l'oxychlorure de phosphore ($POCl_3$) sur la matrice polysaccharidique en présence de NaOH 2N. Avec les conditions réactionnelles décrites, il n'est cependant pas possible d'obtenir des taux de greffage ionique supérieurs à une charge négative pour six résidus glucoses. La Demanderesse a montré que l'efficacité de cette réaction pouvait être améliorée de façon importante en travaillant à basse température et en contrôlant les conditions réactionnelles de température et de pH au cours de la réaction. On obtient ainsi des taux de greffage correspondant à une charge pour 1,5 résidus de glucose.

Dans un autre aspect, l'invention peut être aussi caractérisée dans ce que les étapes a) et b) peuvent être réalisées en même temps grâce à la capacité de réticulation de l'oxychlorure de phosphore. En effet, l'oxychlorure de phosphore a la capacité de former des liaisons phosphodiester entre deux polysaccharides permettant, avec un contrôle adéquate des conditions réactionnelles, de réaliser en même temps la réticulation et l'introduction de charges négatives.

Lorsque l'on souhaite greffer des ligands basiques sur la matrice, un mode de préparation optimal des ligands basiques est décrit ci-après.

Afin d'obtenir le greffage biodégradable de ligands basiques, il est possible d'utiliser l'acide succinique comme composé intermédiaire entre la matrice polysaccharidique et un ligand basique.

La réaction est réalisée en faisant réagir stoéchiométriquement le dichlorure de l'acide succinique avec le composé bifonctionnel. Il se forme un produit de couplage possédant une fonction chlorure d'acide réactive et une fonction basique, amine tertiaire ou quaternaire.

$$CICO\text{-}(CH_2)_2\text{-}COCl + NH_2\text{-}(CH_2)_2\text{-}N(CH_3)_2 \rightarrow CICO\text{-}(CH_2)_2\text{-}CONH\text{-}(CH_2)_2\text{-}N(CH_3)_2$$

La fonction basique permet au réactif d'être soluble dans l'eau et apporte les propriétés d'échange d'ions. La fonction chlorure d'acide permet de réaliser le greffage sur les hydroxyles de la matrice osidique.

Des taux de greffage ionique correspondant à une charge positive pour 1,5 résidus glucose ont pu être obtenus avec ces réactifs, ou bien par exemple une charge pour 3 résidus glucose.

La réaction décrite plus haut peut être utilisée également pour greffer sur la matrice osidique des mono hydroxy ou des mono amino acides sur la matrice osidique.

Dans tous les cas, le taux de greffage ionique de la matrice polysaccharidique est variable, et sera modulé notamment en fonction du type de composé à encapsuler ; on pourra utiliser des vecteurs pour lesquels les taux de greffage sont inférieurs à ceux indiqués ci-dessus comme pouvant être obtenus par les procédés décrits.

On utilisera en particulier des taux de greffage ionique de l'ordre de 1 charge pour 3 résidus glucose.

Les noyaux ioniques peuvent être ultrabroyés par des techniques analogues à celles utilisées pour les matrices polysaccharidiques neutres, comme l'extrusion à haute pression ou les ultra sons. La taille des particules peut être ajustée en réglant les conditions d'ultrabroyage et l'état de dureté du gel, qui dépend du caractère réticulé et de l'état ionique du noyau.

Le séchage des noyaux doit éviter au maximum leur agrégation. Ceci peut être obtenu notamment en réalisant l'étape d) en présence de bicarbonate d'ammonium.

En effet, il est possible de diminuer le degré d'agrégation lors des opérations de séchage par lyophilisation ou atomisation en ajoutant aux suspensions à déshydrater du bicarbonate d'ammonium ($NH_4HCO3$). Ce composé très soluble dans l'eau maintient en effet entre les particules une certaine force ionique qui prévient leur rapprochement et donc leur agrégation éventuelle. Le bicarbonate d'ammonium, étant volatil, est ensuite éliminé lors du séchage et n'interfère pas avec les opérations suivantes. L'utilisation de bicarbonate d'ammonium permet d'obtenir des poudres de particules caractérisées par une densité beaucoup plus faible qu'en l'absence de bicarbonate. La Demanderesse a trouvé que la diminution des phénomènes d'agrégation se traduisait par une augmentation du taux d'acylation des particules et par une plus grande hydrophobie de ces dernières.

La synthèse de la couche lipidique semiperméable est une synthèse régiosélective qui est réalisée par une acylation des noyaux polysaccharidiques en milieu aprotique non solvant des polysaccharides. Parmi les solvants utiles pour cette réaction, la Demanderesse a trouvé qu'il était possible d'utiliser le $CO_2$ à l'état super critique pour effectuer l'étape e).

L'emploi de $CO_2$ à l'état super critique représente une amélioration importante puisque ce composé est non toxique et parfaitement éliminable à l'issue de la réaction, par détente de l'atmosphère réactionnelle.

D'autre part, la Demanderesse a trouvé qu'il est possible de diminuer la densité de la couche lipidique par une acylation ménagée. Le contrôle de la réaction d'acylation peut être réalisé soit par le contrôle de la stoéchiométrie des réactifs, soit par le contrôle de la cinétique réactionnelle.

La Demanderesse a de plus trouvé, que notamment dans le cas des noyaux polysaccharidiques de très petite taille ( < 100nm), l'homogénéité du greffage lipidique pouvait être améliorée en procédant à deux ou plusieurs étapes d'acylation f) successives séparées par des étapes de réhydratation et de séchage e).

Les phénomènes d'agrégation sont, en effet, plus difficiles à éliminer dans le cas des petites particules ; une agrégation résiduelle va entraîner une acylation non homogène de la surface de la particule, conduisant à la présence de parties de surface des noyaux polysaccharidiques acylées et non acylées.

Lorsque de telles particules sont mises en suspension dans l'eau, les parties lipidiques s'associent entre elles permettant la réexposition des parties non acylées.

Un deuxième cycle d'acylation permet alors d'acyler les parties des surfaces des noyaux polysaccharidiques n'ayant pas été acylées lors du premier cycle.

Dans un des aspects préférés de la présente invention, le procédé de synthèse de vecteur particulaire est caractérisé en ce qu'après l'étape e) et avant l'étape f), on inclut dans le noyau des substances à activité biologique.

Un des inconvénients des BVSM décrits dans le brevet original était la possibilité de dériver le produit encapsulé par des acides gras lorsque la réaction d'acylation était réalisée après l'encapsulation.

La Demanderesse a trouvé qu'il est possible de faire pénétrer les principes actifs à l'intérieur du noyau polysaccharidique ionique au travers de la couche semi perméable et d'éviter ainsi la dérivatisation des principes actifs. Une fois que les principes actifs ont franchi la couche lipidique semi perméable, ils sont stabilisés et maintenus à l'intérieur du noyau par l'établissement de liaisons coulombiennes entre leurs charges et les charges de la matrice polysaccharidique.

De plus, des rendements d'encapsulation pratiquement quantitatifs peuvent être obtenus en utilisant une méthode qui consiste à hydrater progressivement un mélange principe actif, avec les noyaux acylés obtenus à l'issue de l'étape e). L'hydratation étant soit par de l'eau, soit par un tampon, soit par un mélange eau/alcool inférieur.

Il est possible que l'encapsulation soit favorisée par les concentrations saturantes en principes actifs ainsi obtenues et par le flux du liquide vers la partie interne des particules induit par leur hydratation.

Dans le cas où la substance à activité biologique est un composé non ionique, le procédé selon l'invention peut être caractérise en ce qu'on greffe, de façon réversible, une charge ionique sur ladite substance avant son inclusion dans le noyau.

En effet, la Demanderesse a trouvé qu'il est possible d'encapsuler des principes actifs polaires non ioniques à l'intérieur de noyaux ioniques en leur greffant de façon réversible une charge ionique. Nous avons trouvé que ce greffage réversible pouvait être réalisé notamment par la réaction des hydroxyles des principes actifs avec le monochlorure de l'acide succinique. Il se forme ainsi un monosuccinate du principe actif présentant une charge négative. Le principe actif original est ensuite retrouvé par l'action de la succinylestérase.

$$ROH + Cl\text{-}CO\text{-}(CH_2)_2\text{-}COOH \rightarrow R\text{-}O\text{-}CO\text{-}(CH_2)_2\text{-}COOH$$

Dans un des modes de mise en oeuvre du procédé selon l'invention, celui-ci peut être caractérisé en ce que l'étape f) est effectuée par dispersion des noyaux acylés obtenus à l'issue de l'étape e), dans lesquels est éventuellement inclus un principe actif, dans un milieu lipidique renfermant des triglycérides et des phospholipides, puis traitement par une lipase. Le caractère hydrophobe des noyaux à couche lipidique semi perméable est suffisant pour leur permettre de se disperser dans des environnements lipidiques comme des triglycérides. Ces dispersions peuvent être comparées à des émulsions eau/huile stabilisées.

Dans un autre des modes de mise en oeuvre du procédé selon l'invention, celui-ci peut être caractérisé en ce que, pour les noyaux de très petite taille (< 50 nm), l'étape f) est effectuée par dispersion des noyaux acylés obtenus à l'issue de l'étape e), dans lequel est éventuellement inclus un principe actif, dans une solution aqueuse de détergent dialysable, dont la molarité est supérieure à la concentration micellaire critique.

La Demanderesse a trouvé qu'il était possible de mélanger la suspension obtenue avec une dispersion de composés amphiphiles comme les phospholipides ou le cholestérol dans la même solution de détergent et d'obtenir les BVSM par dilution rapide de la suspension pour amener le détergent à une molarité inférieure à la CMC, suivie d'une étape de dialyse extensive pour éliminer le détergent.

La Demanderesse a montré notamment qu'il est possible d'établir de manière stable une couche phospholipidique autour des noyaux possédant une couronne lipidique semiperméable.

L'association de cette couche phospholipidique avec les noyaux acylés a été mise en évidence par l'analyse chromatographique des BVSM correspondant, et la parfaite suposition des profils chromatographiques de marqueurs fluorescents greffés de manière covalente sur le noyau ou associés aux phospholipides.

La Demanderesse a montré que les noyaux acylés pouvaient de plus être incorporés aux formulations des composés huileux et notamment aux émulsions. Le système obtenu est ainsi l'équivalent d'une émulsion triple eau/huile/eau.

La Demanderesse a, de plus, trouvé que le traitement d'une émulsion huile/eau, dont la partie huileuse est composée d'un mélange de triglycérides à courte chaîne, de phospholipides et de noyaux acylés, par une lipase conduisait après hydrolyse enzymatique des triglycérides et dialyse à l'obtention de BVSM composés de noyaux acylés entourés de phospholipides.

Les vecteurs particulaires selon la présente invention peuvent être utilisés pour incorporer tout type de molécule chimique active tel que cela a été décrit dans les brevets cités précédemment.

Il peut s'agir par exemple de principes actifs pharmaceutiques et/ou cosmétiques telle que la kératine qui est incorporée dans le noyau.

Les exemples suivants sont destinés à illustrer l'invention et mettent en évidence certains types de molécules qui peuvent être incorporées dans les vecteurs selon l'invention.

## EXEMPLE 1 : PREPARATION DE MATRICES POLYSACCHARIDIQUES BIODEGRADABLES

Dans un réacteur de 5 l, on solubilise 500 g d'amylopectine (Roquette, Lille, France) dans un litre de NaOH 2N. Lorsque la solution est bien homogène, on introduit 28 g d'épichlorydrine (Fluka, Suisse) correspondant à 0,1 équivalent/résidu glucose. Après la fin de l'addition, la préparation est encore homogénéisée 1 h puis laissée reposée 8 h. La préparation d'amidon polymérisé est ensuite amenée à pH 7 par addition d'acide acétique 2 N puis dispersée grossièrement à l'aide d'un broyeur à hélice. Le gel obtenu est ensuite filtré sur Büchner et lavé plusieurs fois avec de l'eau distillée jusqu'à l'élimination de tous les sels et des sous produits de la réaction. On obtient après lyophilisation 450 gr (90 %) de gel réticulé.

## EXEMPLE 2 : DEGRADATION ENZYMATIQUE DU GEL D'AMIDON RETICULE

1 g d'amidon réticulé selon l'exemple 1 est dispersé dans 50 ml de tampon phosphate pH6,9 et mis en présence de 200 unités d'amylase de Bacillus (Sigma). La réaction est agitée pendant une heure à 20°C puis la réaction est stoppée par un chauffage à 90°C pendant 2 min. Le mélange réactionnel est devenu limpide et les produits de réaction sont analysés par HPLC sur une colonne C18 avec une phase mobile entièrement acqueuse et une détection par indice de réfraction. Les résultats obtenus sont comparés à ceux obtenus avec de l'amidon non réticulé et montrent qu'il n'y a pas apparition d'oligomères de taille supérieure à 10.

## EXEMPLE 3 : PREPARATION DE MATRICE POLYSACCHARIDIQUE IONIQUE GREFFEE PAR DE L'ACIDE SUCCINIQUE

### a) Préparation du monochlorure de l'acide succinique : $Cl-CO-(CH_2)_2-COOH$

50 g d'acide succinique sont dispersés dans 50 ml d'une solution anhydre de THF et de DMF (90/10) maintenue à 0°C. On ajoute progressivement, sous agitation, la quantité stoechiométrique de dichlorure de l'acide succinique (66 g). La réaction s'accompagne d'un dégagement de chaleur et le mélange réactionnel est refroidit énergiquement pour maintenir la température en dessous de 0°C. La réaction est poursuivie jusqu'à la dissolution totale de l'acide succinique en suspension. Simultanément à la dissolution de l'acide succinique, on observe la précipitation progressive du monochlorure de l'acide succinique.

Après la fin de la réaction, le monochlorure est précipité complètement par addition d'éther de pétrole anhydre (100 ml) et séparé du mélange réactionnel par filtration sur Büchner. Après lavage du précipité par 100 ml d'éther de pétrole anhydre et séchage au dessicateur sous pression réduite, on obtient 80 g de monochlorure pur (rdt 70 %).

### b) Caractérisation du monochlorure de l'acide succinique

Analyse élémentaire

Valeurs calculées expérimentales :

| | | |
|---|---|---|
| C | 35 % | 36 % |
| H | 3,6 % | 3,4 % |
| O | 35 % | 36,5 % |
| Cl | 26 % | 24,1 % |

Spectre Infra rouge : bandes à 3200 cm-1(s) (OH), 1800 (s) (COCl)

**Caractérisation du monochlorure de l'acide succinique par la formation de l'acide N-(phényl) succinamyloïque :**

$$(C_6H_5)-NH-CO-(CH_2)_2-COOH$$

100 mg de monochlorure de l'acide succinique sont dissous dans 2 ml d'acétonitrile anhydre. On ajoute, à température ambiante, 1,5 équivalents d'aniline (102 mg) et la réaction est maintenue agitée pendant 30 minutes. On ajoute alors 10 ml d'acide sulfurique 0,005 M et on extrait la solution par de l'éther éthylique (3X 3 ml). Les phases étherées sont rassemblées et lavées par de l'eau jusqu'à neutralité puis séchées sur MgS04 anhydre. L'éther est ensuite filtré, évaporé sous pression réduite pour donner 90 mg d'acide N-(phényl) succinamyloïque.

L'analyse chromatographique est réalisée sur une colonne 5 um C8, 3,6 cm X 4,6 mmID avec une détection UV à 254 nm. La colonne est développée par un gradient linéaire entre une phase mobile TFA 5 mM et une phase mobile TFA 5 mM/acétonitrile (30/70). Le chromatogramme indique la présence d'un seul pic caractérisé par un K' de 13,5. On n'observe pas de pics correspondant à l'élution de l'aniline ni du dérivé dianilide de l'acide succinique.

Spectre Infra rouge : bandes à 3300 cm-1 (s) (OH), 1770 cm-1 (s) (COOH), 1650 cm-1 (s) (CON).

Analyse élémentaire

Valeurs calculées expérimentales :

| C | 62,5 % | 64 % |
| N | 7,3 % | 6,9 % |
| H | 5,2 % | 5,7 % |

## c) Greffage de l'acide succinique sur la matrice d'amidon réticulé

100 g d'amidon réticulé préparés selon l'exemple 1, sont dispersés dans 1 litre d'une solution NaCl 2 M et refroidit à 0°C. Le monochlorure de l'acide succinique (85,3 g) est alors ajouté, sous forme de poudre, progressivement à la dispersion agitée doucement et maintenue à pH 6,5 et en dessous de 0°C. Après la fin de l'addition, la réaction est encore agitée une heure à 0°C puis 2 H à température ambiante. Le mélange réactionnel est ensuite acidifié à pH2 par addition d'HCl 2 N et agité une heure. Le gel ionique obtenu est séparé du milieu réactionnel par filtration sur Büchner et lavé plusieurs fois par de l'eau distillée jusqu'à neutralité. On obtient après lyophilisation 110 gr (rdt 78 %) de gel ionique. Le taux de greffage ionique est mesuré par titration du gel par une solution NaOH 0,1 M avec la phénophtaléine comme indicateur. On obtient une charge négative pour 2,5 résidus glucoses.

## EXEMPLE 4 : PREPARATION DE MATRICES POLYSACCHARIDIQUES GREFFEES PAR DE L'ACIDE PHOSPHORIQUE

100 g d'amidon réticulé obtenu selon l'exemple 1 sont dispersés dans une solution de NaCl 2 M ; pH 13,5 et refroidit à 0°C. On ajoute progressivement l'oxychlorure de phosphore (114 ml, 104 g) en maintenant le pH à 13,5 et la température en dessous de 0°C. Après la fin de l'addition, le mélange réactionnel est agité encore deux heures en le laissant revenir à température ambiante. Le mélange est ensuite acidifié à pH2 par ajout d'HCl 2 N, agité une heure, filtré sur Büchner et lavé avec de l'eau distillée jusqu'à neutralité. On obtient après lyophilisation, 110 gr (rdt 90 %) de gel ionique. Le taux de greffage obtenu est déterminé par titration par de la soude 0,1 N avec la phénolphtaléine comme indicateur. On trouve une charge négative pour 1,5 résidus glucoses.

## EXEMPLE 5 : PREPARATION DE MATRICE POLYSACCHARIDIQUE GREFFEE PAR LE N-(2-(DIMETHYLAMINO)ETHYL) SUCCINAMYLATE.

## a) Préparation du chlorure de l'acide N-(2-(diméthylamino)éthyl) succinamyloïque : Cl-CO-(CH$_2$)$_2$-CO-NH-(CH$_2$)$_2$-N(CH$_3$)$_2$)

15,4 g de dichlorure de l'acide succinique sont dissous dans 100 ml d'acétonitrile anhydre et la solution est maintenue sous agitation à 0°C. On ajoute goutte à goutte une solution de 10 g de 2-diméthylamino-éthylamine dans 100 ml d'acétonitrile anhydre. Le milieu réactionnel est encore agité 30 min après la fin de l'addition en maintenant la température à 0°C. Le produit de couplage est alors précipité dans le ballon réactionnel. Lorsque tout le produit est précipité, il est séparé du milieu réactionnel par filtration sur Büchner, lavé plusieurs fois par de l'acétonitrile froid puis séché au dessicateur sous pression réduite pour donner 18 g de produit pur (rdt 84 %).

Analyse élémentaire

Valeurs calculées expérimentales

| | | |
|---|---|---|
| C | 46,7 % | 48 % |
| N | 13,6 % | 12,5 % |
| H | 6,8 % | 7,6 % |

### b) Greffage de l'acide N-(2-(diméthylamino)éthyl)succinamyloïque sur une matrice osidique

100 g d'amidon réticulé selon l'exemple 1 sont dispersés dans un litre de NaCl 2 M maintenue sous agitation à pH 6,5 et à 0°C. On ajoute alors progressivement 63,4 g de chlorure de l'acide N6(2-(diméthylamino) éthyl)succinamylique. Lorsque tout le chlorure d'acide est ajouté, on laisse agiter encore 2 h à 0°C. Le gel ionique obtenu est ensuite filtré sur Büchner, lavé à l'eau distillé et lyophilisé pour donner 105 g de matrice basique (rdt 83 %). Le taux de greffage obtenu est déterminé par l'analyse du taux d'azote présent dans le gel (3,6 %). Ce taux de greffage correspond à une charge positive pour 4 résidus glucoses.

### EXEMPLE 6 : OBTENTION DE PARTICULES POLYSACCHARIDIQUES IONIQUES DE PETITE TAILLE

### a) particules greffées par l'acide succinique

100 gr de gel ionique obtenu selon l'exemple 3 sont dispersés dans 4 litres d'eau distillée et homogénéisés pendant 1 H par un homogénéisateur Rannie (type industrie 12,SI) à une pression de 1000 Bars et un débit de 80 l/h.

L'analyse de la taille des particules obtenues est effectuée au moyen d'un nanosizer Coulter N4 et indique que 90 % des particules ont un diamètre inférieur à 50 nm.

Les particules de très petite taille sont ensuite séchées par lyophilisation ou par atomisation en présence de bicarbonate d'ammonium (50 gr/l). Après séchage, la taille des particules est contrôlée à nouveau au nanosizer après dispersion des particules dans l'eau. Les résultats obtenus sont identiques aux valeurs obtenues avant le séchage. Le taux de greffage ionique est également identique.

### b) particules greffées par l'acide phosphorique

100g du gel phosphorylé obtenu selon l'exemple 4 sont dispersés dans 10l d'eau distillée et homogénéisés au moyen d'un homogénéisateur Rannie 12-51 H. La pression d'homogénéisation est de 900 bars et le débit de 80 l/h.

On obtient une suspension fluide de nanoparticules de polysaccharides réticulés acides dont la taille, mesurée par un nanosizer Coulter N4MD, est centrée autour de 20nm. Les nanoparticules sont alors séchées par lyophilisation en, présence de 50g/l de bicarbonate d'ammonium.

### EXEMPLE 7 : REALISATION DE LA COUCHE LIPIDIQUE SEMIPERMEABLE

10 gr de particules de 50 nm obtenues selon l'exemple 6 (a) sont dispersés dans 30 ml de dichlorométhane. On ajoute 0,8 gr de chlorure d'acide palmitique. Un réfrigérant muni d'une garde au carbonate de potassium est adapté sur le flacon réactionnel et la réaction est agitée énergiquement à reflux pendant une nuit. Le dichlorométhane est ensuite évaporé avec un évaporateur rotatif et le résidu est lavé plusieurs fois par de l'éthanol puis séché sous vide. On obtient 10,3 gr de particules acylées (rdt 100 %). Le taux d'acides gras greffés mesure après saponification des particules est de 5 %. Avec les mêmes conditions d'acylation, le taux d'acides gras greffés sur des particules identiques mais séchées sans bicarbonate d'ammonium est de 3 %.

### EXEMPLE 8 : REALISATION DE LA COUCHE LIPIDIQUE SEMIPERMEABLE PAR ACYLATION MULTIPLE

10 g de particules de 20 nm obtenues selon l'exemple 6 (b) sont dispersées dans 30 ml de dichlorométhane. On ajoute 1,7 g de chlorure d'acide palmitique. Un réfrigérant muni d'une garde au carbonate de potassium est adapté sur le flacon réactionnel et la réaction est agitée énergiquement à reflux pendant une nuit. Le dichlorométhane est ensuite évaporé avec un évaporateur rotatif et le résidu est lavé plusieurs fois par l'éthanol puis séché sous vide. On obtient 10,3 g de particules acylées (rdt. 98 %). Le taux d'acides gras greffés mesure après saponification des particules est de 5 %. Avec les mêmes conditions d'acylation, le taux d'acides gras greffés sur des particules identiques mais séchées sans bicarbonate d'ammonium est de 3 %.

10 g des particules acylées obtenues sont remises en suspension sous forte agitation dans 1 litre d'eau. Une fois que la dispersion est' homogène, les particules sont à nouveau séchées en présence de bicarbonate d'ammonium selon l'exemple 6.

Les particules sont dispersées dans 30 ml de dichlorométhane et réacylées suivant le protocole décrit précédemment. On obtient, après lavage, 9,5 g de particules (rdt. 94 %) avec une taux d'acides gras mesuré à 6 %.

Un troisième cycle réactionnel, comprenant toutes les étapes déjà décrites (hydratation, lyophilisation, réacylation) conduit à 9 g de particules acylées (rdt. total 88 %) avec un taux d'acide gras de 6,5 %.

## EXEMPLE 9 : CARACTERISATION DE LA COUCHE LIPIDIQUE

### A1 - Préparation de BVSM blancs

10 mg de coeurs acylés phosphates 20 nm préparés selon l'exemple 8 sont dispersés dans 1 ml d'Octyl Glucopyranoside OGP 50 mM (Fluka). On les mélange sous ultrasons à une solution de 5 mg de Lécithines de jaune d'oeuf purifiées (Sigma) et de Cholestérol (Sigma) 80/20 dispersée dans 1 ml d'OGP 50 mM. Cette préparation est ensuite diluée brutalement jusqu'à 10 mM sous ultrasons puis dialysée extensivement pendant 48 heures.

### A2 - Préparation de BVSM fluorescents marqués à la Rhodamine

### a - Préparation de coeurs acylés fluorescents marqués à la Rhodamine

50 mg de coeurs acylés phosphates 20 nm préparés selon l'exemple 8 sont dispersés dans 1 ml de Bicarbonate de sodium (Sigma) 100 mM pH 10. On rajoute 0,5 mg d'Isothiocyanate de Rhodamine B (Sigma) solubilisés dans du Diméthyl formamide (SDS), soit 1 % de Rhodamine par rapport au poids de noyaux acylés. Après 12 heures d'agitation à température ambiante, plusieurs lavages à l'éthanol sont effectués afin d'éliminer la Rhodamine n'ayant pas réagi. Les coeurs acylés sont ensuite séchés par lyophilisation.

### b - Etablissement du feuillet phospholipidique

### * A partir de coeurs fluorescents

100 mg de coeurs acylés fluorescents sont dispersés dans 2 ml d'Octyl Glucopyranoside OGP (Fluka) 50 mM. On apporte ensuite à cette suspension 5 mg d'un mélange de Lécithines de jaune d'oeuf purifiées (Sigma) et de Cholestérol (Sigma) 80/20 dispersés dans 1 ml d'OGP 50 mM, soit 50 % de mélange phospholipidique par rapport au poids de noyaux acylés. Cette solution est ensuite diluée brutalement jusqu'à 10 mM sous ultrasons puis dialysée extensivement pendant 48 heures. Deux autres préparations sont également effectuées en utilisant respectivement 20 mg et 30 mg de mélange phospholipidique, soit 200 et 300 % de phospholipides par rapport au poids de noyaux acylés.

### * A partir de phospholipides fluorescents

Les BVSM sont prérarés de manière identique mais avec des coeurs acylés non fluorescents et en introduisant 1 % de phospholipides marqués à la Rhodamine (Molecular Probe) dans le mélange Lécithines de jaune d'oeuf purifiées - Cholestérol.

### c - Préparation de liposomes fluorescents témoins

5 mg de mélange de Lécithines de jaunes d'oeuf - Cholestérol (80/20) contenant 1 % de Phospholipides marqués à la Rhodamine sont dispersés dans 1 ml d'OGP 50 mM. La solution est diluée brutalement jusqu'à 10 mM sous ultrasons puis dialysées extensivement pendant 48 heures.

### B1 - Analyse de la taille des BVSM

L'analyse de la taille des BVSM blancs est effectuée à l'aide d'un nanosizer Coulter N4 et indique que 99 % des particules ont un diamètre de 20 nm (+/- 2 nm).

Cette mesure reste inchangée après 3 mois de conservation à 4°C.

## B2 - Analyse CLHP par perméation de gel

Les BVSM sont détectés par fluorescence (280 nm excitation - 580 nm émission) , sur colonne TSK G 6000 PW, en solvant Tris 10 mM, NaCl 120 mM.

On observe pour les BVSM à coeurs fluorescents à 50 % de phospholipides, un pic correspondant à un volume d'élution de 7,5 ml.

L'analyse des BVSM à 50 % de phospholipides fluorescents permet d'obtenir un pic ayant exactement le même volume d'élution, 7,5 ml.

L'analyse des liposomes fluorescents permet de mettre en évidence un pic ayant un volume d'élution de 9,9 ml, nettement différent du pic des BVSM.

L'analyse des BVSM à 200 et 300 % de phospholipides, permet de mettre en évidence deux pics, l'un à 7,5 ml, l'autre à 9,9 ml. Le pic à 9,9 ml présente une intensité qui augmente avec le pourcentage de phospholipides.

Les résultats indiquent clairement que les phospholipides sont bien associés aux coeurs acylés. Ils indiquent de plus qu'il existe un optimum de phospholipides pour l'établissement de la couche amphiphile, et qu'au delà on observe des liposomes correspondant aux phospholipides surnuméraires.

On constate de plus, que le profil chromatographique des BVSM est inchangé après plusieurs semaines, alors que celui des liposomes évolu en quelques jours, ce qui semble indiquer une plus grande stabilité des BVSM.

## EXEMPLE 10 CHARGEMENT DE LA BUTIROSINE DANS LES BVSM A NOYAUX IONIQUES SUCCINYLES ET A COUCHE LIPIDIQUE SEMIPERMEABLE

La butirosine est un antibiotique de la famille des aminoglycosides. C'est une molécule constituée d'un aminocyclitol lié par des liaisons glycosidiques à des sucres aminés. Il s'agit donc d'un produit très polaire et basique soluble uniquement dans l'eau. Son poids moléculaire est de 556.

On prépare d'abord des noyaux acylés acides de 50 nm selon l'exemple 7. On mélange à l'état sec 1 g de noyaux acylés avec 1 g de butirosine base (Park-Davis). Le mélange est en suite hydraté très progressivement par ajout d'eau distillée. Le mélange est maintenu constamment agité et à 50°C. On ajoute ainsi 10 ml d'eau en laissant le mélange revenir à température ambiante et on laisse agiter encore 2 h.

La suspension obtenue est ensuite lyophilisée. Le résidu sec est dispersé dans 5 ml d'éthanol et ajouté goutte à goutte a une suspension de liposomes unilammellaires (1,5 g de lécithines de jaune d'oeuf purifiées dans 50 ml d'eau distillée). Après sonication au bain pendant 1/2 h, la suspension est ultrafiltrée (point de coupure 7500 Daltons) et la butirosine libre présente dans l'ultrafiltrat est dosée par HPLC. Les résultats indiquent la présence de 50 mg de butirosine dans l'ultrafiltrat, ce qui correspond à un rendement d'incorporation de 95 % et un taux d'incorporation de 95 % en poids de butirosine par rapport au poids du coeur acylé.

## EXEMPLE 11 : CHARGEMENT DE LA BUTIROSINE DANS LES BVSM A NOYAUX IONIQUES PHOSPHORYLES ET A COUCHE LIPIDIQUE SEMIPERMEABLE.

Dans un premier temps, des noyaux acylés acides de 20 nm sont préparés selon l'exemple 8. On mélange ensuite, 50 mg de noyaux acylés avec 25 mg de butirosine base (Park-Davis) dilués dans 1 ml d'eau distillée. Le mélange est maintenu constamment agité à température ambiante, pendant une nuit.

La suspension obtenue est dispersée en présence d'Octyl-d-Glucopyranoside (OGP) (Fluka) jusqu'à une molarité finale de 50 nM et est ajoutée goutte à goutte à une solution de phospholipides (50 mg d'un mélange de lécithines de jaune d'oeuf purifiés / cholestérol (80/20) w/w, dispersés dans 10 ml d'OGP 50 mM). Après sonification au bain pendant 10 minutes, cette solution est brutalement diluée sous Ultra-Sons jusqu'à une molarité de 5 mM en OGP puis, ultrafiltrée (point de coupure : 30 000 daltons). L'analyse de taille effectuée au nanosizer (Coulter N4 SD) indique que 99 % de ces BVSM ont un diamètre de 20 nm (+/-2 nm).

La butirosine libre présente dans le filtrat est dosée par microbiologie. La concentration de l'antibiotique est déterminée par la mesure de l'aire d'inhibition de la croissance de Bacillus subtilis (ATCC 6633). Les résultats indiquent la présence de 2,5 mg de butirosine libre dans l'ultrafiltrat, ce qui correspond à un rendement d'incorporation de 90% de butirosine et à un taux d'incorporation de 45 % en poids de butirosine par rapport au poids de coeurs acylés.

## EXEMPLE 12 : CHARGEMENT DE LA PEROXYDASE DE RAIFORT DANS LES BVSM A NOYAUX ACIDES ET A COUCHE LIPIDIQUE SEMIPERMEABLE

La peroxydase de raifort est une enzyme basique possédant un poids moléculaire de 40.000 Daltons. On utilise les noyaux acylés acides préparés selon l'exemple 7. Un g de noyaux acylés est mélangé à l'état sec avec 1,5 g de peroxydase (Fluka). Le mélange est agité et hydraté très progressivement par ajout d'eau distillée. La température est maintenue à 40°C. On ajoute ainsi 10 ml d'eau distillée et on laisse agiter encore 2h à 40°C. On ajoute alors 10 ml d'une

EP 0 542 969 B1

suspension de 400 mg d'octyl glucoside et de 1,5 g de lécithine de jaune d'oeuf purifiée et le mélange résultant est soniqué pendant 1/2 h au bain en maintenant la température en dessous de 30°C. La suspension obtenue est ensuite dialysée pendant 24 h à 4°C contre de l'eau distillé de façon à éliminer l'octyl glucoside. La préparation est ensuite ultrafiltrée avec une membrane possédant un point de coupure à 100.000 Daltons. La peroxydase libre est dosée dans l'ultrafiltrat par la méthode de Bradford et par dosage enzymatique. Les résultats par les deux méthodes indiquent la présence de 45 mg de péroxydase libre soit un rendement d'encapsulation de 97 % et un taux de chargement de 145 % par rapport au poids de noyaux acylés.

## EXEMPLE 13 PREPARATION DE NOYAUX ACYLES BASIQUES DE 50 nm

Les noyaux basiques sont préparés par le greffage de l'acide N-(2-(diméthylamino)éthyl)succinamyloïque selon l'exemple 5. 200 g de matrice PS basique sont dispersées dans 2 l d'eau distillée et ultrabroyés à haute pression (800 bars) avec un homogénéisateur Rannie pour donner des particules de 50 nm. Ces particules sont atomisées en présence de bicarbonate d'ammonium (50 g/l) pour donner 130 g de poudre sèche qui sont suspendus dans 400 ml de dichlorométhane. On ajoute alors 16 g de chlorure d'acide oléique et on laisse sous agitation pendant 24 h à température ambiante. Les particules acylées obtenues sont alors séparées du milieu réactionnel par centrifugation et lavées avec du dichlorométhane puis avec de l'éthanol. La suspension éthanolique finale est évaporée sous vide à froid pour donner 125 g de noyaux basiques acylés. Le taux de greffage d'acides gras est mesuré par dosage de l'acide oléique libre après saponification des particules. On trouve un taux de 4,5 %.

## EXEMPLE 14 : CHARGEMENT D'UN NUCLEOTIDE : L'ADENOSINE 5' MONOPHOSPHATE (AMP)

L'AMP est une molécule acide d'un poids moléculaire de 347. Le chargement est donc effectué sur des noyaux basiques.

50 mg de noyaux basiques préparés selon l'exemple 13 sont mélangés avec 10 mg d'AMP (Fluka) dilués dans 1 ml d'eau distillée. L'incorporation est réalisée sous agitation, à température ambiante pendant 3 heures. La suspension obtenue est ensuite dispersée en présence d'Octyl-d-Glucopyranoside (OGP) jusqu'à une moralité finale de 50 mM et est ajoutée goutte à goutte, à une solution de phospholipides (50 mg d'un mélange de lécithines de jaune d'oeuf purifiées / cholestérol (80/20) w/w, dispersés dans 10 ml d'OGP 50 mM). Après sonication au bain pendant 10 minutes, cette solution est brutalement diluée sous Ultra-Sons jusqu'à une molarité de 5 mM en OGP puis, ultrafiltrée (point de coupure : 30 000 daltons). L'analyse de taille effectuée au nanosizer (Coulter N4 SD) indique que 99 % de ces BVSM ont un diamètre de 50 nm (+/-3 nm).

L'AMP libre est dosée par spectrophotométrie. Les résultats obtenus indiquent une valeur de 0,5 mg d'AMP soit un rendement d'incorporation de 95 % et un taux de chargement de 19 % par rapport au poids de noyaux acylés.

## EXEMPLE 15 : CHARGEMENT D'UNE MOLECULE NON IONIQUE, LE STACHYOSE

Le stachyose est un tétrasaccharide de structure Gal 1--> 6 Gal 1--> 6Glc 1--> 2 Fru. C'est un composé dépourvu de charge électrique et très polaire. La structure du stachyose ne lui permet donc pas d'être encapsulé dans un des compartiments polaires ou lipophiles des BVSM. Nous avons réalisé l'encapsulation en greffant à la molécule de stachyose une fonction ionique acide couplée à un hydroxyle du stachyose par une fonction ester hydrolysable enzymatiquement.

### a) Préparation du dérivé acide du stachyose

1 g de stachyose est dissous dans 5 ml d'une solution aqueuse maintenue sous agitation à 0°C et à pH 6,5 par un pHstat. On ajoute progressivement à cette solution 135 mg de monochlorure d'acide succinique préparé selon l'exemple 3. Après la fin de l'addition, on laisse encore agiter pendant 2 h en laissant revenir à température ambiante. Le mélange réactionnel est appliqué au sommet d'une colonne de perméation de gel G25 et élué par de l'eau distillée. Les fractions contenant l'oligosaccharide (testé par la méthode de Dubois) sont rassemblées et lyophilisées pour donner 0,85 g de stachyose modifié. Le taux de greffage ionque est mesuré par titration avec la soude 0,1 N et correspond a une charge ionique pour 4,2 sucres donc environ une charge par molécule de stachyose.

### b) Chargement du stachyose modifié

50 mg de noyaux basiques acylés préparés selon l'exemple 10 sont mélangés intimement à l'état sec avec 25 mg de stachyose modifié. Le mélange est ensuite hydraté très progressivement avec 1 ml d'eau distillée sous agitation constante et à température ambiante pendant 3 heures. La suspension obtenue est dispersée en présence d'Octyl Glucopyranoside OGP (Fluka) jusqu'à une molarité finale de 50 mM. Elle est ensuite ajoutée goutte à goutte à une préparation de Phospholipides (50 mg de Lécithines de jaune d'oeuf purifiées - Cholestérol (80/20) dans 10 ml d'OGP 50 mM. Après sonication pendant 10 minutes au bain, la préparation est diluée brutalement jusqu'à 10 mM. Après sonication pendant 10 minutes au bain, la préparation est diluée brutalement jusqu'à 10 mM sous ultrasons puis ultrafiltrée et le stachyose libre est dosé par la méthode de Dubois. Les résultats indiquent une vlaeur de 2,5 mg de stachyose dans l'ultrafiltrat, soit un rendement d'encapsulation de 90 % et un taux de chargement de 45% par rapport au poids de noyaux acylés.

L'analyse de la taille est effectuée à l'aide d'un nanosizer Coulter N4 et indique que 98 % des particules ont un diamètre de 50 nM (+/- 3 nm).

## EXEMPLE 16 : PREPARATION DE BVSM A PARTIR D'UNE DISPERSION DE NOYAUX ACYLES DANS UNE SOLUTION DE TRIGLYCERIDES

15 mg de noyaux polysaccharidiques (50 nm), préparés selon l'exemple 7, sont dispersés dans un mélange de 30 mg de lécithines de jaune d'oeuf purifiée et de 250 mg de tributyrine. Le mélange est repris dans 10 ml de tampon Tris-maléate 0,1 pH 7,2 et la suspension est homogénéisée au Vortex et par agitation magnétique. L'ensemble est placé au bain marie à 37°C et 25 mg de lipase (Type VII, C. Cylindrae, Sigma) sont ajoutés. L'acide butyrique libéré est neutralisé par addition de NaOH 0,01 M au moyen d'un pHstat maintenant le pH à 7,2. Après 30 mn d'incubation, la suspension s'est éclaircie et la mesure des tailles (Coulter N4SD, Coultronics) indique une population de taille centrée autour de 50 nm. La suspension est ensuite transférée dans un boudin de dialyse (Cut-off 12.000-14.000, Spectrapor), placée dans un litre de tampon tris-maléate 0,01 M, pH 7,2 et laissée une nuit à dialyser. L'analyse, par chromatographie en phase gazeuse, de la solution contenue dans le boudin de dialyse indique l'absence de tributyrine et d'acide butyrique.

## EXEMPLE 17 : PREPARATION DES NOYAUX ACYLES DANS LE $CO_2$ SUPERCRITIQUE

0,3 g de noyaux polysaccharidiques ioniques, préparés selon l'exemple 6, sont placés dans un réacteur en saphire de 10 ml éprouvé à 150 bars. On ajoute 60 mg de chlorure d'acide oléique et on pressurise le réacteur à 100 bars avec du $CO_2$ anhydre. La réaction est ensuite maintenue sous agitation 12 h à 40°C. Le $CO_2$ est ensuite détendu, le résidu est lavé plusieurs fois par de l'éthanol puis séché sous vide à basse température. On obtient 0,25 g de particules acylées. Le taux d'acide gras greffés mesuré après saponification des particules est de 6 %.

## EXEMPLE 18 : PREPARATION DES MATRICES HYDROPHILES PAR RETICULATION D'OLIGOSACCHARIDES PAR L'OXYCHLORURE DE PHOSPHORE

Dans un ballon de 500 ml on introduit 50 g de dextrine 10 (Fluka) (poids moléculaire 1620) que l'on dissout dans 45 ml d'eau contenant 1 g de borohydrure de sodium.

La réaction est agitée pendant deux heures à température am biante jusqu'à la réduction totale des sucres réducteurs terminaux afin d'éviter les réactions indésirables d'énolisation des polysaccharides en milieu basique.

Une fois la température stabilisée à 0°C, on ajoute 24 g d'oxychlorure de phosphore (POCl3, 0,15 M) goutte à goutte, sous agitation énergique. En même temps, 57 ml de NaOH 10 M sont ajoutés de façon à ce que l'addition des réactifs se fasse de façon simultanée.

Après la fin de l'addition dès réactifs, le mélange réactionnel est encore agité doucement pendant 1 heure et puis neutralisé à pH 7 par addition d'acide acétique.

Le gel ainsi obtenu est ensuite dispersé grossièrement à l'aide d'un broyeur à hélice, filtré sur un büchner et lavé plusieurs fois à l'eau distillée jusqu'à élimination des sels et des sous-produits de la reaction.

Le gel est finalement précipité à l'éthanol et séché sous pression réduite pour obtenir 40 g de dextrine réticulée (80 % rendement).

La titration de 1 g de gel réticulé à l'aide d'un titrimètre automatique (Titroprocesseur Methrom 682) révèle un volume de neutralisation de 1,8 mEq/g correspondant à la première acidité du phosphate greffé et de 1,2 mEq/g correspondant à la deuxième acidité. Ceci indique un taux de réticulation de 0,6 mEq de fonctions phosphodiester par gramme de gel réticulé.

## EXEMPLE 19 : INCORPORATION DE KERATINE DANS DES BVSM A NOYAUX CATIONIQUES DE 200nm

La kératine est la protéine de structure des couches superficielles de la peau. Elle est disponible sous forme d'hydrolysats partiels solubles de poids moléculaire moyen d'environ 100.000 et de point isoélectrique compris entre 5,0 et 7,0.

**\* Préparation d'une matrice polysaccharidique cationique**

Dans un réacteur de 30l, on solubilise 2kg d'amidon de maïs (Roquette, Lille, France) dans 5l de NaOH 2N. Lorsque la solution est homogène, on introduit 1kg de chlorure de glycidyl triméthylammonium, dissout dans 500ml d'eau, soit 0,5 équivalents/résidu glucose. La préparation est encore homogénéisée 2h puis laissée au repos pendant 10h et enfin neutralisée à pH 7 par addition d'acide chlorhydrique 2N. Le gel est ensuite lavé plusieurs fois avec de l'eau distillée jusqu'à élimination de tous les sels et sous-produits de la réaction à l'aide d'une essoreuse centrifuge.

**\* Obtention de particules de 200nm**

1kg du gel précédemment préparé est dispersé dans 40l d'eau distillée et homogénéisé pendant 4h par un homogénéisateur Rannie Lab 12-51 (APV Rannie, Copenhague, Danemark) à une pression de 500 bars et un débit de 120l/h.

L'analyse de la taille des particules obtenues est effectuée au moyen d'un nanosizer Coulter N4 et indique que la dispersion est centrée sur une taille moyenne de 200nm.

Ces partiucles sont ensuite séchées au moyen d'un atomiseur APV Lab 1 en présence de bicarbonate d'ammonium (50 g/l).

**\* Réalisation de la couche lipidique périphérique**

200g de particules séchées sont dispersées dans 2,5l de dichlorométhane. On ajoute 30g de chlorure de l'acide palmitique. La réaction est agitée pendant une nuit. Le dichlorométhane est ensuite filtré et le résidu est lavé plusieurs fois avec du dichlorométhane, puis de l'éthanol et enfin séché sous vide. Le taux d'acides gras greffés, mesure après saponification, est de 0,8%.

**\* Chargement de la kératine dans les particules polysaccharidiques cationiques**

200g de noyaux polysaccharidiques acylés sont réhydratés lentement par 1l d'une solution à 400g/l d'hydrolysat soluble de kératine (Croda) sous agitation lente, puis additionnés de 2l d'eau distillée. Au bout de 8h, on ajoute 10g de phosphatidylcholine de soja hydrogénée (Nattermann, France) et on laisse agiter 1h. La suspension est injectée dans 17l d'eau distillée et homogénéisée dans un homogénéisateur Rannie Lab 12-51 (APV Rannie, Copenhague, Danemark) à la pression de 300 bars pendant 1h au débit de 120l/h.

La suspension ainsi obtenue est analysée pour déterminer la taille des particules au moyen d'un nanosizer Coulter N4. La dispersion est centrée autour de 160nm. Une ultrafiltration de la suspension, suivie d'un dosage des protéines totales par la méthode de Bradford, montre la présence de 80g de kératine dans l'ultrafiltrat, soit un rendement d'encapsulation de 80% et un taux de chargement de 160% par rapport au poids de noyaux acylés.

**EXEMPLE 20 : PREPARATION DE BVSM CARACTERISES PAR UN NOYAU CENTRAL D'AMIDON CORETICULE AVEC DE LA KERATINE**

200g d'amidon soluble (Prolabo, Paris, France) et 20g d'hydrolysat soluble de kératine sont dispersés dans 3l de NaOH 2N dans un réacteur de 5l. Lorsque la solution est homogène, on ajoute 9,7ml d'épichlorhydrine, correspondant a 0,1 équivalent / résidu glucose. La préparation est homogénéisée 2h puis laissée au repos pendant 10h et enfin neurtralisée à pH 7 par addition d'acide acétique 2N. Le gel est ensuite lavé plusieurs fois avec de l'eau distillée jusqu'à élimination de tous les sels et sous-produits de la réaction à l'aide d'une essoreuse centrifuge. Ce gel polysaccharidique est ensuite dispersé dans 4l d'eau distillée et homogénéisé pendant 4h par un homogénéisateur Rannie Mini Lab (APV Rannie, Copenhague, Danemark) à une pression de 600 bars et un débit de 10l/h.

L'analyse de la taille des particules obtenues est effectuée au moyen d'un nanosizer Coulter N4 et indique que la dispersion est centrée sur une taille moyenne de 210nm.

Une microanalyse élémentaire révèle une proportion d'azote d'environ 1,1% du poids total, soit un rendement de coréticulation kératine-polysaccharide supérieur à 95%.

Ces particiules sont ensuite séchées par atomisation ou par lyophilisation en présence de bicarbonate d'ammonium (50g/l) acylées et dispersées dans le feuillet de phosphatidylcholine de soja hydrogénée selon les protocoles décrits dans l'exemple 19. Les dispersions ainsi obtenues sont centrées autour d'une taille de 190nm.

**EXEMPLE 21 : ETABLISSEMENT D'UN FEUILLET AMPHIPHILE EXTERNE COMPOSE DE CERAMIDES**

Une suspension de 2,5g de céramides (Bio Europe, Toulouse, France) dans 5l d'eau distillée est homogénéisée par un homogénéisateur Rannie à 300 bars pendant 15 minutes à un débit de 10l/h.

50g de noyaux polysaccharidiques acylés, préparés selon l'exemple 19, sont ajoutés progressivement à cette suspension tout en maintenant la pression de 300 bars, puis homogénéisés pendant 3h. La dispersion obtenue est analysée au moyen d'un nanosizer Coulter N4 et la taille moyenne est mesurée à 230nm.

**Revendications**

1. Procédé de synthèse d'un vecteur particulaire, caractérisé en ce que :

   a) on prépare une matrice par réticulation d'un polymère ou d'un oligomère hydrophile biodégradable,

   b) on fixe sur la matrice, des ligands ioniques, avec la condition que les réactifs ne sont pas des chlorures d'acide gras et/ou un anhydride de diacide, afin d'obtenir le noyau du vecteur,

   c) on soumet les noyaux à ultrabroyage pour les amener à une taille comprise entre 10 nm et 10 $\mu$m,

   d) on sèche les noyaux,

   e) on couple chimiquement sur les fonctions réactives à la surface du noyau, des composés lipidiques pour former la première couche,

   f) on introduit des composés amphiphiles en contact hydrophobe avec la première couche afin de former la seconde couche.

2. Procédé selon la revendication 1, caractérisé en ce que les étapes a) et b) sont réalisées simultanément par action de l'acide phosphorique ou l'un de ses dérivés notamment l'oxychlorure de phosphore.

3. Procédé selon la revendication 1, caractérisé en ce qu'à l'étape b) on fixe sur la matrice de l'acide succinique en utilisant le monochlorure d'acide succinique selon la réaction suivante:

$$PS\text{-}OH + Cl\,CO\text{-}(CH_2)_2\text{-}COOH \rightarrow PS\text{-}O\text{-}CO\text{-}(CH_2)_2\text{-}COOH + HCl$$

dans laquelle PS-OH représente la matrice réticulée porteuse d'une fonction OH.

4. Procédé selon l'une des revendications 1 à 3, caractérisé en ce que les composés lipidiques introduits à l'étape e) sont des acides gras.

5. Procédé selon l'une des revendications 1 à 4, caractérisé en ce qû'après l'étape e) et avant l'étape f), on inclut dans le noyau des substances à activité biologique.

6. Procédé selon l'une des revendications 1 à 5, caractérisé en ce que l'étape d) est effectuée en présence de bicarbonate d'ammonium.

7. Procédé selon l'une des revendications 1 à 6, caractérisé en ce que les étapes d) et e) sont répétées plusieurs fois en intercalant une étape d'hydratation.

8. Procédé selon l'une des revendications 1 à 7, caractérisé en ce que l'étape e) est effectuée dans du $C0_2$ à l'état supercritique.

9. Procédé selon l'une des revendications 5 à 8, caractérisé en ce que l'inclusion de la substance à activité biologique est effectuée par mélange de ladite substance avec les noyaux obtenus à l'issue de l'étape e), et hydratation progressive.

10. Procédé selon l'une des revendications 5 à 9, caractérisé en ce que la substance à activité biologique est un composé non ionique et en ce qu'on greffe, de façon réversible, une charge ionique sur ladite substance avant son inclusion dans le noyau.

11. Procédé selon l'une des revendications 1 à 10, caractérisé en ce que l'étape f) est effectuée par dispersion des noyaux acylés obtenus à l'issue de l'étape e), dans lesquels est éventuellement inclus un principe actif, dans un milieu lipidique renfermant des triglycérides et des phospholipides, puis traitement par une lipase.

12. Procédé selon l'une des revendications 1 à 11, caractérisé en ce que l'étape f) est effectuée par une méthode de dialyse de détergent.

13. Vecteur particulaire biodégradable, susceptible d'être obtenu par le procédé selon l'une des revendications 1 à 12, caractérisé en ce qu' il comporte :

- un noyau constitué d'une matrice polysaccharidique ou oligosaccharidique réticulée, sur laquelle sont greffés des ligands ioniques, répartis de façon homogène,
- une première couche lipidique semi-perméable, liée au noyau par des liaisons covalentes,
- une seconde couche de composés amphiphiles, liée à la première couche lipidique par des interactions hydrophobes.

14. Vecteur particulaire selon la revendication 13, caractérisé en ce que les ligands greffés sur la matrice sont des composés acides.

15. Vecteur particulaire selon l'une des revendications 13 et 14, caractérisé en ce que les ligands acides greffés sur la matrice sont choisis parmi l'acide succinique, l'acide phosphorique, l'acide citrique, la glycine, l'alanine, l'acide glutamique, l'acide aspartique ou leurs dérivés.

16. Vecteur particulaire selon la revendication 15, caractérisé en ce que l'acide succinique est greffé à un taux allant jusqu'à une charge pour 1,5 résidus de glucose.

17. Vecteur particulaire selon la revendication 15, caractérisé en ce que l'acide phosphorique est greffé à un taux allant jusqu'à une charge pour 1,5 résidus de glucose.

18. Vecteur particulaire selon la revendication 13, caractérisé en ce que les ligands greffés sur la matrice sont des composés basiques.

19. Vecteur particulaire selon la revendication 18, caractérisé en ce que les ligands greffés sur la matrice sont des composés basiques, fixés à la matrice par l'intermédiaire d'un composé acide.

20. Vecteur particulaire selon l'une des revendications 18 et 19, caractérisé en ce que les ligands greffés sur la matrice sont des composés bifonctionnels comportant une fonction acylable formant une liaison amide ou ester avec un composé acide fixé sur la matrice.

21. Vecteur particulaire selon la revendication 20, caractérisé en ce que le ligand basique est choisi parmi la choline, l'hydroxycholine, le 2-(diméthylamino)éthanol et la 2-(diméthylamino)éthylamine.

22. Vecteur particulaire selon l'une des revendications 18 à 21, caractérisé en ce que le ligand basique est greffé à un taux allant jusqu'à une charge pour 1,5 résidus de glucose.

23. Vecteur particulaire selon l'une des revendications 13 à 22, caractérisé en ce que la matrice est constituée d'un polysaccharide réticulé choisi parmi le dextrane, l'amidon, la cellulose et leurs dérivés.

24. Vecteur particulaire selon l'une des revendications 13 à 23, caractérisé en ce que la matrice est coréticulée avec une protéine ou un peptide

25. Vecteur particulaire selon la revendication 24, caractérisé en ce que la protéine est choisie parmi: la kératine, le collagène, l'élastase, leurs dérivés et leurs analogues.

26. Vecteur particulaire selon l'une des revendications 13 à 25, caractérisé en ce que la première couche lipidique semi-perméable est constituée d'acides gras naturels fixés à un taux variable.

27. Vecteur particulaire selon l'une des revendications 13 à 26, caractérisé en ce que la seconde couche lipidique est constituée de phospholipides.

28. Vecteur particulaire selon l'une des revendications 13 à 27, caractérisé en ce que la seconde couche lipidique est constituée de céramide.

29. Vecteur particulaire selon l'une des revendications 13 à 28, caractérisé en ce que la seconde couche est formée d'un double feuillet de phospholipides, le feuillet interne étant interdigité par les acides gras de la première couche lipidique.

**30.** Vecteur particulaire selon l'une des revendications 13 à 29, caractérisé en ce qu'une molécule à activité biologique est incluse dans le noyau.

**31.** Vecteur particulaire selon la revendication 30, caractérisé en ce que la molécule à activité biologique a un poids moléculaire compris entre 100 daltons et 500 kilodaltons.

**32.** Vecteur particulaire selon l'une des revendications 13 à 31, caractérisé en ce que le noyau à une taille comprise entre 10 nm et 10 µm.

**33.** Procédé de préparation de noyaux polysaccharidiques susceptibles de servir à la synthèse de vecteurs selon l'une des revendications 13 à 32, caractérisé en ce que :

a) on prépare une matrice par réticulation d'un polymère ou d'un oligomère hydrophile biodégradable,
b) on fixe sur la matrice, des ligands ioniques, avec la condition que les réactifs ne sont pas des chlorures d'acide gras et/ou un anhydride de diacide, afin d'obtenir le noyau du vecteur,
c) on soumet les noyaux à ultrabroyage pour les amener à une taille comprise entre 10 nm et 10 µm.

**34.** Procédé selon la revendication 33, caractérisé en ce que les étapes a) et b) sont réalisées simultanément par action de l'acide phosphorique ou l'un de ses dérivés notamment l'oxychlorure de phosphore.

**35.** Procédé selon l'une des revendications 33 ou 34, caractérisé en ce qu'après l'étape c), on sèche les noyaux.

**36.** Particule polysaccharidique susceptible d'être obtenue par le procédé selon l'une des revendications 33 à 35, caractérisée en ce qu'elle comporte un noyau constitué d'une matrice polysaccharidique ou oligosaccharidique réticulée sur laquelle sont greffés des ligands ioniques répartis de façon homogène, d'une taille comprise entre 10 nm et 10 µm.

**37.** Particule polysaccharidique selon la revendication 36, caractérisée en ce que sa taille est inférieure à 100 nm.

## Claims

**1.** Method of synthesis of a particulate vector, characterized in that:

a) a matrix is prepared by crosslinking a biodegradable hydrophilic polymer or oligomer,
b) ionic ligands are fastened onto the matrix, provided that the reactants are not fatty acid chlorides and/or a diacid anhydride, in order to obtain the nucleus of the vector,
c) the nuclei are subjected to ultragrinding in order to bring them to a size of between 10 nm and 10 µm,
d) the nuclei are dried,
e) lipid compounds are coupled chemically to the reactive functional groups at the surface of the nucleus in order to form the first layer,
f) amphiphilic compounds in hydrophobic contact with the first layer are introduced in order to form the second layer.

**2.** Method according to Claim 1, characterized in that Stages a) and b) are carried out simultaneously by reacting with phosphoric acid or one of its derivatives, especially phosphorus oxychloride.

**3.** Method according to Claim 1, characterized in that, in Stage b), succinic acid is fastened onto the matrix by using succinic acid monochloride according to the following reaction:

$$PS\text{-}OH + Cl\,CO\text{-}(CH_2)_2\text{-}COOH \rightarrow PS\text{-}O\text{-}CO\text{-}(CH_2)_2\text{-}COOH + HCl$$

in which PS-OH represents the crosslinked matrix carrying an OH functional group.

**4.** Method according to one of Claims 1 to 3, characterized in that the lipid compounds introduced in Stage e) are fatty acids.

**5.** Method according to one of Claims 1 to 4, characterized in that, after Stage e) and before Stage f), substances having biological activity are included in the nucleus.

6. Method according to one of Claims 1 to 5, characterized in that Stage d) is carried out in the presence of ammonium bicarbonate.

7. Method according to one of Claims 1 to 6, characterized in that Stages d) and e) are repeated several times, intercalating a hydration stage.

8. Method according to one of Claims 1 to 7, characterized in that Stage e) is carried out in $CO_2$ in the supercritical state.

9. Method according to one of Claims 5 to 8, characterized in that inclusion of the substance having biological activity is carried out by mixing the said substance with the nuclei obtained at the end of Stage e), and progressive hydration.

10. Method according to one of Claims 5 to 9, characterized in that the substance having biological activity is a nonionic compound and in that an ionic charge is reversibly grafted onto the said substance before its inclusion in the nucleus.

11. Method according to one of Claims 1 to 10, characterized in that Stage f) is carried out by dispersion of the acylated nuclei obtained at the end of Stage e), in which nuclei is optionally included an active principle, in a lipid medium containing triglycerides and phospholipids, and then by treatment with a lipase.

12. Method according to one of Claims 1 to 11, characterized in that Stage f) is carried out by a detergent dialysis method.

13. Biodegradable particulate vector, capable of being obtained by the method according to one of Claims 1 to 12, characterized in that it comprises:

   - a nucleus consisting of a crosslinked polysaccharide or oligosaccharide matrix, onto which ionic ligands are grafted with a homogeneous distribution
   - a first semipermeable lipid layer, bonded to the nucleus by covalent bonds,
   - a second layer of amphiphilic compounds, bonded to the first lipid layer by hydrophobic interactions.

14. Particulate vector according to Claim 13, characterized in that the ligands grafted onto the matrix are acidic compounds.

15. Particulate vector according to one of Claims 13 and 14, characterized in that the acidic ligands grafted onto the matrix are chosen from succinic acid, phosphoric acid, citric acid, glycine, alanine, glutamic acid, aspartic acid and their derivatives.

16. Particulate vector according to Claim 15, characterized in that succinic acid is grafted at a degree ranging up to one charge per 1.5 glucose residues.

17. Particulate vector according to Claim 15, characterized in that phosphoric acid is grafted at a degree ranging up to one charge per 1.5 glucose residues.

18. Particulate vector according to Claim 13, characterized in that the ligands grafted onto the matrix are basic compounds.

19. Particulate vector according to Claim 18, characterized in that the ligands grafted onto the matrix are basic compounds, fastened onto the matrix via an acidic compound.

20. Particulate vector according to one of Claims 18 and 19, characterized in that the ligands grafted onto the matrix are bifunctional compounds containing an acylable functional group which forms an amide or ester bond with an acidic compound fastened to the matrix.

21. Particulate vector according to Claim 20, characterized in that the basic ligand is chosen from choline, hydroxycholine, 2-(dimethylamino)ethanol and 2-(dimethylamino)ethylamine.

22. Particulate vector according to one of Claims 18 to 21, characterized in that the basic ligand is grafted at a degree ranging up to one charge per 1.5 glucose residues.

23. Particulate vector according to one of Claims 13 to 22, characterized in that the matrix consists of a crosslinked polysaccharide chosen from dextran, starch, cellulose and their derivatives.

24. Particulate vector according to one of Claims 13 to 23, characterized in that the matrix is co-crosslinked with a protein or a peptide.

25. Particulate vector according to Claim 24, characterized in that the protein is chosen from: keratin, collagen, elastase, their derivatives and their analogues.

26. Particulate vector according to one of Claims 13 to 25, characterized in that the first semipermeable lipid layer consists of natural fatty acids fastened to a variable degree.

27. Particulate vector according to one of Claims 13 to 26, characterized in that the second lipid layer consists of phospholipids.

28. Particulate vector according to one of Claims 13 to 27, characterized in that the second lipid layer consists of ceramide.

29. Particulate vector according to one of Claims 13 to 28, characterized in that the second layer is formed of a double phospholipid lamella, the internal lamella being interdigitated by the fatty acids of the first lipid layer.

30. Particulate vector according to one of Claims 13 to 29, characterized in that a molecule having biological activity is included in the nucleus.

31. Particulate vector according to Claim 30, characterized in that the molecule having biological activity has a molecular weight of between 100 daltons and 500 kilodaltons.

32. Particulate vector according to one of Claims 13 to 31, characterized in that the nucleus has a size of between 10 nm and 10 μm.

33. Method of preparation of polysaccharide nuclei which are capable of being used for the synthesis of vectors according to one of Claims 13 to 32, characterized in that:

   a) a matrix is prepared by crosslinking a biodegradable hydrophilic polymer or oligomer,
   b) ionic ligands are fastened onto the matrix, provided that the reactants are not fatty acid chlorides and/or a diacid anhydride, in order to obtain the nucleus of the vector,
   c) the nuclei are subjected to ultragrinding in order to bring them to a size of between 10 nm and 10 μm.

34. Method according to Claim 33, characterized in that Stages a) and b) are carried out simultaneously by reacting with phosphoric acid or one of its derivatives, especially phosphorus oxychloride.

35. Method according to one of Claims 33 and 34, characterized in that, after Stage c), the nuclei are dried.

36. Polysaccharide particle capable of being obtained by the method according to one of Claims 33 to 35, characterized in that it contains a nucleus composed of a crosslinked polysaccharide or oligosaccharide matrix, onto which ionic ligands are grafted with a homogeneous distribution, with a size of between 10 nm and 10 μm.

37. Polysaccharide particle according to Claim 36, characterized in that its size is less than 100 nm.


**Patentansprüche**

1. Verfahren zur Synthese eines particulären Vektors, dadurch gekennzeichnet, daß man

   a) durch Vernetzung eines biologisch abbaubaren hydrophilen Polymers oder Oligomers eine Matrix herstellt
   b) an der Matrix ionische Liganden fixiert mit der Maßgabe, daß die Reagentien keine Fettsäurechloride und/oder kein Disäureanhydrid sind, unter Bildung des Vektor-Kerns,
   c) die Kerne einer Ultrafeinzerkleinerung unterwirft, um sie auf eine Teilchengröße zwischen 10 nm und 10 μm

zu bringen,
d) die Kerne trocknet,
e) Lipid-Verbindungen an die reaktionsfähigen Funktionen an der Oberflache des Kerns chemisch ankoppelt unter Bildung der ersten Schicht und
f) amphiphile Verbindungen in hydrophobem Kontakt mit der ersten Schicht einführt unter Bildung der zweiten Schicht.

2. Verfahren nach Anspruch 1, dadurch gekennzeichnet, daß die Stufen (a) und (b) gleichzeitig durchgeführt werden unter Verwendung von Phosphorsäure oder einem ihrer Derivate, insbesondere Phosphoroxychlorid.

3. Verfahren nach Anspruch 1, dadurch gekennzeichnet, daß man in der Stufe (b) an der Matrix Bernsteinsäure fixiert unter Verwendung des Monochlorids der Bernsteinsäure nach der folgenden Reaktionsgleichung:

$$PS\text{-}OH + ClCO\text{-}(CH_2)_2\text{-}COOH \rightarrow PS\text{-}O\text{-}CO\text{-}(CH_2)_2\text{-}COOH + HCl$$

in der PS-OH die vernetzte Matrix darstellt, die eine OH-Funktion trägt.

4. Verfahren nach einem der Ansprüche 1 bis 3, dadurch gekennzeichnet, daß es sich bei den in der Stufe (e) eingeführten Lipid-Verbindungen um Fettsäuren handelt.

5. Verfahren nach einem der Ansprüche 1 bis 4, dadurch gekennzeichnet, daß man nach der Stufe (e) und vor der Stufe (f) dem Kern Substanzen mit einer biologischen Aktivität einverleibt.

6. Verfahren nach einem der Ansprüche 1 bis 5, dadurch gekennzeichnet, daß die Stufe (d) in Gegenwart von Ammoniumbicarbonat durchgeführt wird.

7. Verfahren nach einem der Ansprüche 1 bis 6, dadurch gekennzeichnet, daß die Stufen (d) und (e) mehrmals wiederholt werden unter Zwischenschaltung einer Hydratationsstufe.

8. Verfahren nach einem der Ansprüche 1 bis 7, dadurch gekennzeichnet, daß die Stufe (e) in $CO_2$ im superkritischen Zustand durchgeführt wird.

9. Verfahren nach einem der Ansprüche 5 bis 8, dadurch gekennzeichnet, daß die Einarbeitung der Substanz mit biologischer Aktivität erfolgt durch Mischen der genannten Substanz mit den am Ende der Stufe (e) erhaltenen Kernen und fortschreitende Hydratation.

10. Verfahren nach einem der Ansprüche 5 bis 9, dadurch gekennzeichnet, daß die Substanz mit biologischer Aktivität eine nicht-ionische Verbindung ist und daß man auf reversible Weise eine ionische Ladung auf die genannte Substanz aufpfropft, bevor man sie in den Kern einarbeitet.

11. Verfahren nach einem der Ansprüche 1 bis 10, dadurch gekennzeichnet, daß die Stufe (f) durchgeführt wird durch Dispergieren der am Ende der Stufe (e) erhaltenen acylierten Kerne, in denen gegebenenfalls ein Wirkstoff (aktives Prinzip) enthalten ist, in einem Lipid-Medium, das Triglyceride und Phospholipide enthält, und anschließendes Behandeln mit einer Lipase.

12. Verfahren nach einem der Ansprüche 1 bis 11, dadurch gekennzeichnet, daß die Stufe (f) durchgeführt wird unter Anwendung eines Detergens-Dialyse-Verfahrens.

13. Biologisch abbaubarer partikulärer Vektor, der nach dem Verfahren nach einem der Ansprüche 1 bis 12 erhältlich ist, dadurch gekennzeichnet, daß er umfaßt

- einen Kern, bestehend aus einer vernetzten Polysaccharid- oder Oligosaccharid-Matrix, auf die in homogener Verteilung ionische Liganden aufgepfropft sind,
- eine semipermeable erste Lipid-Schicht, die durch kovalente Bindungen an den Kern gebunden ist,
- eine zweite Schicht von amphiphilen Verbindungen, die durch hydrophobe Wechselwirkungen an die erste Lipid-Schicht gebunden ist.

14. Partikulärer Vektor nach Anspruch 13, dadurch gekennzeichnet, daß die auf die Matrix aufgepfropften Liganden saure Verbindungen (Säure-Verbindungen) sind.

**15.** Partikulärer Vektor nach einem der Ansprüche 13 und 14, dadurch gekennzeichnet, daß die auf die Matrix aufgepfropften sauren Liganden ausgewählt werden aus der Gruppe Bernsteinsäure, Phosphorsäure, Zitronensäure, Glycin, Alanin, Glutaminsäure, Asparaginsäure oder ihren Derivaten.

**16.** Partikulärer Vektor nach Anspruch 15, dadurch gekennzeichnet, daß die Bernsteinsäure in einem Grad (Ausmaß) aufgepfropft werden kann, der (das) bis zu einer Ladung pro 1,5 Glucose-Resten geht.

**17.** Partikulärer Vektor nach Anspruch 15, dadurch gekennzeichnet, daß die Phosphorsäure in einem Grad aufgepfropft wird, der bis zu einer Ladung pro 1,5 Glucose-Resten geht.

**18.** Partikulärer Vektor nach Anspruch 13, dadurch gekennzeichnet, daß die auf die Matrix aufgepfropften Liganden basische Verbindungen sind.

**19.** Partikulärer Vektor nach Anspruch 18, dadurch gekennzeichnet, daß die auf die Matrix aufgepfropften Liganden basische Verbindungen sind, die mittels einer sauren Verbindung (Säureverbindung) an der Matrix fixiert sind.

**20.** Partikulärer Vektor nach einem der Ansprüche 18 und 19, dadurch gekennzeichnet, daß die auf die Matrix aufgepfropften Liganden bifunktionelle Verbindungen sind, die eine acylierbare Funktion tragen, die mit einer an der Matrix fixierten sauren Verbindung (Säureverbindung) eine Amid- oder Ester-Bindung bildet.

**21.** Partikulärer Vektor nach Anspruch 20, dadurch gekennzeichnet, daß der basische Ligand ausgewählt wird aus der Gruppe Cholin, Hydroxycholin, 2-(Dimethylamino)ethanol und 2-(Dimethylamino)ethylamin.

**22.** Partikulärer Vektor nach einem der Ansprüche 18 bis 21, dadurch gekennzeichnet, daß der basische Ligand in einem Grad aufgepfropft wird, der bis zu einer Ladung pro 1,5 Glucose-Resten geht.

**23.** Partikulärer Vektor nach einem der Ansprüche 13 bis 22, dadurch gekennzeichnet, daß die Matrix aus einem vernetzten Polysaccharid besteht, das ausgewählt wird aus der Gruppe Dextran, Stärke, Cellulose und ihren Derivaten.

**24.** Partikulärer Vektor nach einem der Ansprüche 13 bis 23, dadurch gekennzeichnet, daß die Matrix zusammen mit einem Protein oder einem Peptid gemeinsam vernetzt ist.

**25.** Partikulärer Vektor nach Anspruch 24, dadurch gekennzeichnet, daß das Protein ausgewählt wird aus der Gruppe Keratin, Collagen, Elastase, ihren Derivaten und ihren Analoga.

**26.** Partikulärer Vektor nach einem der Ansprüche 13 bis 25, dadurch gekennzeichnet, daß die semipermeable erste Lipid-Schicht aus natürlichen Fettsäuren besteht, die in einem variablen Grad (Ausmaß) daran fixiert sind.

**27.** Partikulärer Vektor nach einem der Ansprüche 13 bis 26, dadurch gekennzeichnet, daß die zweite Lipid-Schicht aus Phospholipiden besteht.

**28.** Partikulärer Vektor nach einem der Ansprüche 13 bis 27, dadurch gekennzeichnet, daß die zweite Lipid-Schicht aus Ceramid besteht.

**29.** Partikulärer Vektor nach einem der Ansprüche 13 bis 28, dadurch gekennzeichnet, daß die zweite Schicht aus einer Doppellamelle aus Phospholipiden gebildet ist, wobei die innere Lamelle durch die Fettsäuren der ersten Lipid-Schicht interdigitalisiert ist.

**30.** Partikulärer Vektor nach einem der Ansprüche 13 bis 29, dadurch gekennzeichnet, daß in dem Kern ein Molekül mit biologischer Aktivität eingeschlossen ist.

**31.** Partikulärer Vektor nach Anspruch 30, dadurch gekennzeichnet, daß das Molekül mit biologischer Aktivität ein Molekulargewicht zwischen 100 Dalton und 500 Kilodalton hat.

**32.** Partikulärer Vektor nach einem der Ansprüche 13 bis 31, dadurch gekennzeichnet, daß der Kern eine Teilchengröße zwischen 10 nm und 10 μm hat.

**33.** Verfahren zur Herstellung von Polysaccharid-Kernen, die für die Synthese von Vektoren nach einem der Ansprüche 13 bis 32 verwendbar sind, dadurch gekennzeichnet, daß man

a) durch Vernetzung eines biologisch abbaubaren hydrophilen Polymers oder Oligomers eine Matrix herstellt,
b) an der Matrix ionische Liganden fixiert mit der Maßgabe, daß die Reagentien keine Fettsäurechloride und/oder kein Disäureanhydrid sind, unter Bildung des Vektor-Kerns,
c) die Kerne einer Ultrafeinzerkleinerung unterwirft, um sie auf eine Teilchengröße zwischen 10 nm und 10 $\mu$m zu bringen.

34. Verfahren nach Anspruch 33, dadurch gekennzeichnet, daß die Stufen (a) und (b) gleichzeitig durchgeführt werden unter Verwendung von Phosphorsäure oder einem ihrer Derivate, insbesondere Phosphoroxychlorid.

35. Verfahren nach einem der Ansprüche 33 oder 34, dadurch gekennzeichnet, daß man nach der Stufe (c) die Kerne trocknet.

36. Polysaccharid-Teilchen, wie es nach dem Verfahren nach einem der Ansprüche 33 bis 35 erhältlich ist, dadurch gekennzeichnet, daß es einen Kern aufweist, der besteht aus einer vernetzten Polysaccharid- oder Oligosaccharid-Matrix, auf die ionische Liganden in homogener Verteilung aufgepfropft sind, mit einer Teilchengröße zwischen 10 nm und 10 $\mu$m.

37. Polysaccharid-Teilchen nach Anspruch 36, dadurch gekennzeichnet, daß seine Teilchengröße unter 100 nm liegt.